(19) Europäisches Patentamt
European Patent Office
Office européen des brevets

(11) Veröffentlichungsnummer: **0 055 382**
**B1**

(12) # EUROPÄISCHE PATENTSCHRIFT

(45) Veröffentlichungstag der Patentschrift: **31.07.85**

(21) Anmeldenummer: **81109249.3**

(22) Anmeldetag: **29.10.81**

(51) Int. Cl.⁴: **C 12 P 7/56**, C 12 P 39/00, A 23 C 21/02, A 23 K 1/22, C 12 M 1/36

(54) **Verfahren zur Herstellung von Ammoniumlactat enthaltenden Mischungen und Vorrichtung zur Durchführung des Verfahrens.**

(30) Priorität: **29.12.80 DE 3049381**

(43) Veröffentlichungstag der Anmeldung: **07.07.82 Patentblatt 82/27**

(45) Bekanntmachung des Hinweises auf die Patenterteilung: **31.07.85 Patentblatt 85/31**

(84) Benannte Vertragsstaaten: **AT CH DE FR GB LI NL**

(56) Entgegenhaltungen:
**DE - A - 2 731 877**

**APPLIED AND ENVIRONMENTAL MICROBIOLOGY, Band 32, Nr. 6, Dezember 1976, American Society for Microbiology, Washington D.C. (US) C.A. REDDY et al.: "Bacterial Fermentation of Cheese Whey for Production of a Ruminant Feed Supplement Rich in Crude Protein", Seiten 769-776**
**JOURNAL OF DAIRY SCIENCE, Band 41, Nr. 7, Juli 1958, Champaign, Ill. (US) D.R. ARNOTT et al.: "A method for manufacturing a high-nitrogen-low-lactose product from whey", Seiten 931-941**
**MICROBIOLOGY ABSTRACTS, Section A, Industrial and applied microbiology, Band 11, Nr. 3, März 1976, Seite 25, Zusammenfassung Nr. 11A1228, London (GB) J.D. EFSTATHIOU et al.: "Growth and preservation parameters for preparation of a mixed species culture concentrate for cheese manufacture"**

(73) Patentinhaber: **Schanze, Rudolf, Friedenstrasse 43, D-8034 Unterpfaffenhofen (DE)**

(72) Erfinder: **Schanze, Rudolf, Friedenstrasse 43, D-8034 Unterpfaffenhofen (DE)**

(74) Vertreter: **Kraus, Walter, Dr. et al, Patentanwälte Kraus Weisert & Partner Thomas-Wimmer-Ring 15, D-8000 München 22 (DE)**

(56) Entgegenhaltungen: (Fortsetzung)

EP 0 055 382 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

In der DE-OS 2 731 877 des gleichen Anmelders wird ein Verfahren zur Herstellung von Ammoniumlactat enthaltenden Mischungen aus wäßrigen Milchverarbeitungsrückständen beschrieben, das dadurch gekennzeichnet ist, daß man zu den Milchverarbeitungsrückständen oder Gemengen, die Milchsäure und/oder Lactose und Milchsäurebildner enthalten oder zu denen man Milchsäurebildner zugeben kann, ohne weitere Vorbehandlung, gegebenenfalls unter Erwärmen oder Kühlen und Bewegen, Ammoniak in solcher Rate zugibt, daß der pH-Wert unter 6,0 gehalten wird; Ammoniak bis zu einem pH-Wert von höchstens 6,5 zugibt, nachdem die gewünschte Menge an Ammoniumlactat gebildet worden ist und gegebenenfalls anschließend das erhaltene Reaktionsgemisch in an sich bekannter Weise bis auf einen Feststoffgehalt von maximal 85% konzentriert.

Bei dem oben beschriebenen Verfahren wird 1 Molekül Milchzucker (Lactose) in 2 Moleküle Milchsäure gespalten und diese Milchsäure reagiert mit Ammoniak unter Bildung von Ammoniumlactat, wie es in der folgenden Gleichung dargestellt wird:

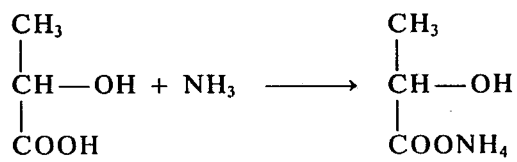

Die vorliegende Erfindung betrifft eine Verbesserung des oben beschriebenen Verfahrens sowie eine Vorrichtung zur Durchführung des Verfahrens.

In der US-PS 2 904 437 wird ein Verfahren zur Behandlung von Molke beschrieben, bei dem die Molke biochemisch unter Zugabe von Ammoniak oxidiert wird, während der pH-Wert im Bereich von 5,6 bis 6 gehalten wird. Nachdem die Lactose in die Milchsäure überführt worden ist, wird das oxidierte Material konzentriert und auf einen pH-Wert im Bereich zwischen etwa 7 und 8 mit Ammoniak neutralisiert. Das dabei erhaltene Produkt ist neutral oder schwach alkalisch und riecht nach Ammoniak. Seine Haltbarkeit ist nur begrenzt, da aus dem Produkt Ammoniak entweicht. Bei diesem bekannten Verfahren wird als Kultur L.bulgaricus verwendet. Als Substrat kann nur Molke aus der Käseherstellung eingesetzt werden.

D. Arnott, S. Patton und E. Kesler (Anim. nutr. 78/1973, Seite 931) beschreiben ein Verfahren von der Ammoniierung von Molke aus den Produkten von Cheddarkäse mit Lactobacillus bulgaricus. Bei diesem bekannten Verfahren erfolgt die Ammonisierung kontinuierlich oder diskontinuierlich.

C. A. Reddy, H. E. Henderson und M. D. Erdman beschreiben in Appl. and environmental Mikrobiol., Dezember 1976, Band 32, Seite 769, ausführliche Ergebnisse über die bakterielle Fermentierung von Käsemolke, wobei ein Tierfutter erhalten wird. Auch hier wird Käsemolke, und zwar Lab- oder Süßmolke, und Molke aus den Produkten von Cottage Cheese ammonisiert. Es werden Reinkulturen von Lactobacillus bulgaricus, Stamm 2217, verwendet, wobei die Fermentation bei einem pH-Wert von 5,5 bei ca. 43° C als bevorzugt und die Endneutralisation bei einem pH-Wert von 6,8 erfolgen. Ferner wurde gefunden, daß im Substrat ein Lactoseanteil bis 7% zu normalem Verlauf führt, während bereits bei 11,5% Lactosegehalt im Substrat Rest von unammonisierter Lactose verbleiben. Bis 7% Lactose im Substrat wird die gesamte Lactose bis auf vernachlässigbare Reste in Ammoniumlactat umgewandelt.

Die oben beschriebenen Verfahren besitzen besonders für die Großtechnik Bedeutung. Die Großtechnik hat jedoch in den letzten Jahren eine starke Strukturveränderung erfahren, so daß die oben beschriebenen Verfahren nicht mehr zufriedenstellend großtechnisch verwendet werden können.

In früheren Jahren haben Molken aus der Produktion von Käse eine wirtschaftliche Verwendung gefunden. Inzwischen haben neuere Erkenntnisse in der Tierernährung, insbesondere bei Milchaustauschern, dazu geführt, daß hochwertige Molken aus der Verlabung von Milch, aber auch saure Molken, in zunehmendem Maße anstelle von Muttermilch bei Kälbern, Ferkeln und Fohlen eingesetzt werden können und daß man mit diesen Stoffen auch so profitable Preise erreicht. Weiterhin werden Molken neuerdings zur Rückgewinnung des hochwertigen Molkenproteins und der anschließenden Gewinnung von Milchzucker verwendet. Dazu verwendet man moderne Techniken, wie die Hyperfiltration, welche als Sammelbegriff für Methoden der Membrantrennung in Form der inversen Osmose, der Ultrafiltration und Elektrodialyse angesehen werden kann.

So fallen heute zahlreiche Restprodukte und Rückstände an, die als Permeat, Restfiltrat oder auch kurz als Koppelprodukte bezeichnet werden und die einen stark verminderten Proteingehalt und teils mehr, teils erhöhte Milchzuckergehalte in der restlichen Trockensubstanz aufweisen.

In der folgenden Tabelle I sind die Zusammensetzungen der Trockenmassen von Koppelprodukten der Milchzerlegung, insbesondere der Rückstände, aufgeführt.

Tabelle I

| | Bandbreiten der Inhaltsstoffe in Prozent, bezogen auf Gesamttrockenmasse (=TS) | | | | | |
|---|---|---|---|---|---|---|
| | Protein | NPN*) | Lactose | Milch-säure | Asche/ Mineral | nativ/ TS |
| Molke aus Speisequark, Frischkäse | 10—14 | 1—3 | 50—70 | 5—25 | 9—11 | 5—7 |
| milchsaure Molken | 11—14 | 1—4 | 60—75 | 5—10 | 8—10 | 5—6,5 |
| Molke aus Thermoquark | 6—10 | 1—4 | 65—75 | 5—25 | 8—12 | 4,5—6 |
| Salzwasser aus El-Dial. | — | 10—20 | 10—40 | 5—15 | 30—60 | 2—5 |
| Molken enteiweißt aus Membrantrenn. Permeate | 5—10 | 1—3 | 70—90 | 1—10 | 5—12 | 5—15 |
| Molken entzuckert aus Lactoseprod. | 15—25 | 2—4 | 35—50 | 1—5 | 20—30 | 30—60 |
| Milchzuckerwasser aus Lactoseprodukt. | 4—10 | 2—5 | 80—90 | ? | 5—10 | 35—65 |

*) NPN = Non Protein Nitrogen, stickstoffhaltige Verbindungen, die nicht mehr als Protein oder Eiweiß bezeichnet werden können, niedermolekular.

Aus dieser Tabelle ist erkennbar, daß der Lactosegehalt dieser Produkte sehr hoch ist, so daß sie nicht mehr gemäß den oben beschriebenen, bekannten Verfahren in Ammoniumlactat überführt werden können.

Die oben beschriebenen Produkte sind grundsätzlich stark umweltbelastend und haben einen hohen Sauerstoffbedarf bei Einleitung in Abwässer und sind daher in zunehmendem Maße kostenbelastend. Andererseits sind sie in ihren Eigenschaften so, daß eine weitere Verwertung an technischen und wirtschaftlichen Schwierigkeiten scheitert oder zu Produkten führt, die im Markt nicht untergebracht werden können.

Bei den oben beschriebenen, bekannten Verfahren wird außerdem als Kultur Lactobacillus bulgaricus verwendet. Dieser Stamm besitzt den Nachteil, daß die Fermentation nur innerhalb eines sehr engen pH-Bereiches und innerhalb eines sehr engen Temperaturbereichs durchgeführt werden kann. Weiterhin wird der Stamm leicht durch Inhaltsstoffe im Ausgangsmaterial verunreinigt und inaktiviert. Dadurch ist das Verfahren sehr anfällig. Wird der bevorzugte Temperaturbereich oder pH-Bereich verlassen, kommt die Fermentation zum Erliegen. Dies ist für den technischen Betrieb, insbesondere für die laufende Produktion, sehr nachteilig, da dann das ganze Verfahren abgebrochen und die Produkte entweder verworfen oder gereinigt werden müssen. Verwendet man nur eine einzige Kultur, so sind die Verfahrensparameter sehr beschränkt, und dies ist für den großtechnischen Betrieb nachteilig.

Der vorliegenden Anmeldung liegt die Aufgabe zugrunde, ein Verfahren zur Verfügung zu stellen, mit dem Produkte der Milchindustrie, die auch einen Lactosegehalt über 7% aufweisen, ammonisiert werden können. Erfindungsgemäß sollen die Produkte, wie sie oben teilweise aufgeführt wurden und die heute verworfen werden einer nützlichen Verwendung zugeführt werden. Insbesondere sollen Produkte, die stark umweltbelastend sind, auf wirtschaftliche Weise einer nutzvollen Endverwendung zugeführt werden. Weiterhin sollen vorhandene technische Einrichtungen, wie Vorratstanks, Pumpen und Armaturen, weitgehend genutzt werden können. Außerdem soll erfindungsgemäß auf Kulturen zurückgegriffen werden, die üblich sind, und das Personal soll von der Prozeßführung weitgehend entlastet werden.

Die Ammonisierung soll in einer Form erfolgen, die leicht zu handhaben ist und keine krassen Neutralisationvorgänge einführt, wie zum Beispiel das Einleiten von $NH_3$ in Gasform. Der Bedarf an Nebeninvestitionen soll auf einem Minimum gehalten werden.

Erfindungsgemäß soll ein Verfahren zur Verfügung gestellt werden, gemäß dem innerhalb eines großen Temperaturbereiches und innerhalb eines großen pH-Bereiches gearbeitet werden kann und bei dem Verunreinigungen, die möglicherweise im Ausgangsprodukt enthalten sind, nicht den Verfahrensablauf zum Erliegen bringen.

Überraschenderweise wurde gefunden, daß diese Aufgabe gelöst werden kann, wenn man anstelle von Reinkulturen Mischkulturen verwendet und eine spezielle Apparatur einsetzt, die dem Verfahren angepaßt ist.

Gegenstand der Erfindung ist somit ein Verfahren zur Herstellung von Ammoniumlactat enthaltenden Mischungen aus wäßrigen Milchverarbeitungsrückständen gemäß dem Oberbegriff von Patentanspruch 1, das dadurch gekennzeichnet ist, daß man als Milchsäurebildner Mischkulturen verwendet.

Die Erfindung betrifft weiterhin eine Vorrichtung zur Durchführung des Verfahrens.

Das erfindungsgemäße Verfahren besitzt den Vorteil, daß sämtliche, in der Milchverarbeitung anfallende Produkte, auch solche mit hohem Lactosegehalt und solche, die stark verunreinigt sind, ammonisiert werden können und auf wirtschaftliche und einfache Weise in Ammoniumlactat überführt werden können, ohne daß eine Kontamination beobachtet wird und die Umsetzung unterbrochen wird. Weiterhin können vorhandene technische Einrichtungen, wie Vorratstanks, Pumpen und Armaturen, genutzt werden.

Man kann auf Kulturen zurückgreifen, die üblich sind, und das Personal ist von der Prozeßführung weitgehend entlastet. Die Ammonisierung erfolgt auf einfache Weise, und der Bedarf an Neuinvestitionen wird auf einem Minimum gehalten.

Überraschenderweise wurde gefunden, daß man bei Verwendung von Mischkulturen die oben beschriebenen Nachteile von Lactobacillus bulgaricus vermeiden kann, d. h., daß die Ammoniumlactatbildung ohne Störungen abläuft. Die Mischkulturen besitzen je nach Betriebsverhältnissen verschiedene Bandbreiten an Substrat und umfassen verschiedene Temperatur- und pH-Bereiche. Sie können sich auch an die jeweiligen Substratverhältnisse durch Verschiebungen in ihrer Population flexibel anpassen.

Erfindungsgemäß verwendet man Mischkulturen, die im Handel erhältlich sind und für die Herstellung von beispielsweise Sauerrahmbutter, Käse und Speisequark angeboten werden. Überraschenderweise wurde gefunden, daß diese Kulturen auch vorteilhaft für die spezielle Milchsäurebildung, für die sie nicht gedacht sind, eingesetzt werden können. Es besteht jedoch ein grundsätzlicher Unterschied in der Verwendung. Bei den oben genannten üblichen Anwendungen werden die Kulturen in Milchen eingesetzt, und es wird abgewartet, bis nach unterschiedlichen Zeiten der gewünschte End-pH-Wert erreicht wird. Lediglich der Temperaturbereich wird kontrolliert. Man unterscheidet bei solchen Mischkulturen Mischkulturen, die im normalen Temperaturbereich, im mesophilen oder thermophilen Bereich (d. h. bei mittlerer Temperatur oder bei erhöhter Temperatur) wirken. Bei der Herstellung von Sauerrahmbutter, Käse oder Speisequark wird dieser Temperaturbereich, der vom Hersteller der Kulturen angegeben wird, verwendet.

Die im Handel erhältlichen Mischkulturen für die oben genannten Anwendungsgebiete eignen sich auch als Mischkulturen bei der vorliegenden Erfindung.

Von den im Handel erhältlichen Mischkulturen für die oben genannten Verwendungen sind bei dem erfindungsgemäßen Verfahren einige besonders gut geeignet. Um festzustellen, ob eine im Handel erhältliche Mischkultur erfindungsgemäß verwendet werden kann, werden Laborvorversuche durchgeführt. Solche Laborvorversuche sind dem Fachmann geläufig, und daher sollen sie hier nur kurz erläutert werden. In Abhängigkeit von dem zu behandelnden Produkt und der Temeratur, bei der das Produkt anfällt, werden von den im Handel erhältlichen Mischkulturen solche Mischkulturen herausgegriffen, die in dem Temperaturbereich, in dem das Produkt in der Molkerei oder im Milchverarbeitungsbetrieb anfällt, wirken. In anderen Worten, man trifft eine Auswahl, ob man eine bei normaler Temperatur wirkende Mischkultur oder mesophile oder thermophile Mischkulturen verwendet. Die Hersteller von Mischkulturen geben für die Kulturen immer Richtwerte an. In einem Vorversuch wird dann das zu behandelnde Produkt mit unterschiedlichen Mengen der Kulturen in Mengen von 0,5 bis 1,0, maximal 2% Anteil (Anzahl der Mikroorganismenpopulation $10^8$ $^9/g$) bei unterschiedlichen pH-Bereichen zwischen 3,0 bis 5,5 auf den Fermentationsverlauf geprüft. Beispielsweise wird ein Versuch mit 1,0 Gew.-% Kultur bei einem pH-Wert im Bereich von $3,5 \pm 0,3$, einem pH-Wert im Bereich von $4,5 \pm 0,3$ und einem pH-Wert im Bereich von $5,5 \pm 0,3$ durchgeführt. In diesen unterschiedlichen pH-Bereichen wird der Fermentationsablauf geprüft. Die Anlaufzeit, die Aktivität hinsichtlich der Milchsäurebildung bzw. der $NH_3$-Verbrauch in den unterschiedlichen pH-Bändern werden getestet und beurteilt. Die gewonnenen Erkenntnisse über Kultureinsatzmenge, Aktivität und Milchsäurebildung, bezogen auf den besten pH-Bereich, werden dann verwendet, um die Mischkultur auszuwählen, die am besten geeignet ist. Es ist vom wirtschaftlichen Standpunkt aus wesentlich, daß so wenig wie möglich Kultur eingesetzt werden muß, damit das Verfahren wirtschaftlich durchgeführt werden kann. Die bei den Vorversuchen erhaltenen Ergebnisse werden dann für die Voreinstellung des Steuergeräts verwendet.

Die in der Praxis der großtechnischen Anwendung erfolgende Veränderung dieser Werte auf optimalen Effekt werden dann vom Prozeßrechenteil des Steuergeräts bei Bedarf nachkorrigiert.

In der folgenden Tabelle sind einige Bezugsquellen für Mischkulturen aufgeführt. Weiterhin sind die Handelsbezeichnungen von Kulturen angegeben, die für das erfindungsgemäße Verfahren besonders gut geeignet sind. Schließlich ist noch die Art der Kultur angegeben.

Tabelle I

| Bezugsquelle | Bezeichnung der Kultur im Handel | Kulturtyp (grundsätzlich Mischkulturen) |
|---|---|---|
| Miles GmbH<br>Lyoner Str. 32<br>6000 Frankfurt 71<br>(0611-666027) | CR 7<br><br><br>THY 42 | mesophil, 28—33° C<br>heterofermentativ<br><br>thermophil, 42—43° C<br>heterofermentativ |
| Laboratorium Wiesby GmbH & Co<br>Postfach 1366<br>Gotteskoogstr. 40,<br>2260 Niebüll<br>(04661-715/6) | Joghurt 231 | thermophil, 42—43° C<br>heterofermentativ |
| Laboratorium Dr. Drewes KG<br>Bergstraße 3<br>Postfach 68<br>3370 Seesen a. Harz<br>(05381-2291) | SSK | thermophil, 42—43° C<br>heterofermentativ |
| Probiotic AG/SA,<br>CH-5200 Brugg (Schweiz)<br>(004156-410251) | Strcc. faeeium<br>Cernelle, SF68 | thermophil, 37—39° C<br>fast homofermentativ |
| | gekoppelt mit einer der oberen vier Kulturen einzusetzen | |

Anhand solcher Vorversuche ist es für den Fachmann einfach, unter den verfügbaren im Handel erhältlichen Mischkulturen solche auszuwählen, die für das erfindungsgemäße Verfahren geeignet sind, d. h. bei denen die Milchzuckerspaltung in Milchsäure innerhalb geeigneter Zeiten abläuft.

Vorzugsweise verwendet man bei dem erfindungsgemäßen Verfahren ein Gemisch aus Kulturen, die im oberen pH-Bereich wirken, und Kulturen, die im unteren pH-Bereich wirken.

Grundsätzlich stehen im oberen pH-Bereich die Streptokokken, wie Str. lactis, Str. faecalis, Str. salivarius, Str. cremoris, Str. thermophilus, Str. diacetylis, Str. faecium, und im unteren pH-Bereich Stäbchen- und Langstäbchenbakterien, wie der zitierte Lbc. bulgaricus, Lbc. lactis, Lbc. helveticus, Lbc. acidophilus und Lbc. delbrückii, zur Verfügung. Diese Gemische können als weitere Bakterien Leuconosatocarten (mesenteroides, cremoris), Streptobacter, Betabakterien, Lbc. fermentum und viridescens, gegebenenfalls Bifidobacterium bifidus, Propionobacter enthalten.

Die Kulturen unterscheiden sich hinsichtlich ihrer Temperaturbeständigkeit, Säurebeständigkeit und auch hinsichtlich der von ihnen erzeugten organischen Säuren, die über die Produktion von Milchsäure hinaus auch zu Essigsäure, Diacetyl, Propionsäure, Zitronensäure usw. bzw. zu den entsprechenden Ammoniumsalzen führen können.

Die Bildung dieser Säuren ist nicht unerwünscht, da ein breites Spektrum physiologisch positiver, ammonisierbarer Säuren zu einer Verbesserung des Geschmacks und damit der Bekömmlichkeit des Futters über unterschiedliche Aromen und unterschiedliche stützende physiologische Eigenschaften führt.

Durch die Mischkulturen dieser unterschiedlichen Stämme wird erreicht, daß der Prozeß bei unterschiedlichen Temperaturen, die je nach Betrieb technisch zwischen 25 und 60° für das Substrat liegen können, durchgeführt werden kann. Dies ist von besonderem Vorteil, da bei den bekannten Verfahren immer bestimmte, genaue Temperaturen eingehalten werden mußten. Das erfindungsgemäße Verfahren kann überraschenderweise auch bei unterschiedlichen pH-Bereichen, die ebenfalls zwischen einem pH-Wert von 3,0 bis 6,5 liegen können, durchgeführt werden. Die pH-Werte hängen von der Menge und der Art der Rückstände ab. Es ist ein großer Vorteil des erfindungsgemäßen Verfahrens, daß die pH-Werte variieren können. Bei dem erfindungsgemäßen Verfahren kann weiterhin die Konzentration im Substrat unterschiedlich sein und zwischen 5,0 und 15 Gew.-% Trockenmasse, bezogen auf 100 Gewichtsteile Substrat, liegen. Das erfindungsgemäße Verfahren erlaubt somit eine optimale Anpassung an alle praktischen Bedingungen, da sämtliche kritischen Bedingungen, wie die Temperatur, der pH-Wert und die Zusammensetzung der Trockenmasse, variierbar sind.

Erfindungsgemäß kann man als Milchverarbeitungsrückstand Süßmolke, milchsaure Molke aus Speisequark oder Frischkäse, Molke aus Thermoquark, enteiweißte Molken aus der Membranabtrennung, entzuckerte Molken aus der Lactoseproduktion, Salzwasser aus der Elektrodialyse, Permeate, Milchzuckerwasser aus der Lactoseproduktion, Buttermilch oder Rückstände, die bei der Milchzerlegung mit Hilfe moderner Verfahren, wie zum Beispiel Ultrafiltration, Umkehrosmose, Elektrodialyse, anfallen, oder ihre Gemische verwenden. Es ist ein besonderer Vorteil des erfindungsgemäßen Verfahrens, daß man auch sehr verdünnte und teilweise verunreinigte Produkte, die man in der Vergangenheit in das Abwasser eingeleitet hat, verwenden kann.

Es ist allgemein bekannt, daß biochemische Verfahren sehr empfindlich sind und auf kleinste Schwankungen im pH- und Temperaturbereich reagieren. Es ist daher erforderlich, daß das erfindugnsgemäße Verfahren so geführt wird, daß sämtliche Schwankungen sofort ausgeglichen werden. Es ist einleuchtend, daß das üblicherweise zur Verfügung stehende Personal solche Prozeßschwankungen nicht immer ausreichend verfolgen kann, und daher muß nach einem Weg gesucht werden, unabhängig vom Personal die Steuerung des Verfahrens durchzuführen.

Eine Vorrichtung für eine solche Prozeßsteuerung muß daher in der Lage sein:

(1) die Bandbreiten der pH-Steuerung sicher zu regeln und im Optimum zu halten;
(2) die Bandbreiten der Temperatur sicher zu regeln;
(3) die Dosierung von Ammoniak in das Substrat gezielt vorzunehmen;
(4) Ausfälle im Prozeß zu registrieren und zu kompensieren;
(5) die quantitative Ammonisierung zu einem bestimmten Punkt zu führen;
(6) den Prozeßverlauf umzusetzen in eine optimale Produktion von Milchsäure und nachfolgend von Ammoniumlactat.

Dabei ist zu berücksichtigen, daß je nach Lage im Betrieb diese Steuerung sowohl im Inneren der Betriebsräume als auch im Freien durchführbar sein muß.

Zu diesem Zweck wurde ein Gerät entwickelt, das in der Lage ist, unabhängig vom Aufstellungsort, sowohl im Freien als auch im Innenraum stationiert zu werden. Dies wird dadurch erreicht, daß das Gerät so kompakt gebaut ist, daß es alle Steuerungsprozesse in sich vereinigt und durch Rollen so beweglich gehalten wird, daß es an vorhandene Einrichtungen, wie Tanks, Pumpen, Leitungen und dergleichen herangeführt und angeschlossen werden kann.

Gegenstand der Erfindung ist somit eine Vorrichtung zur Durchführung des Verfahrens, die gekennzeichnet ist durch einen Tank, eine Steuerungseinrichtung, Leitungen zwischen dem Tank und dem Steuergerät, Pumpen, einen Wärmeaustauscher, einen Ammoniakvorratstank, Leitungen zur Ammoniakzufuhr, eine Netzzuführung und Druckluftleitungen für den Wärmeaustauscher.

Gegenstand der Erfindung ist weiterhin eine Steuerungseinrichtung, die gekennzeichnet ist durch ein Gehäuse, ein Durchlaufrohr für die Milchverarbeitungsrückstände mit einem Schauglas, zwei Einrichtungen zur pH-Messung, zwei Einrichtungen zur Temperaturmessung, eine Dosierpumpe, eine Zufuhrleitung für Ammoniak mit einem Durchflußmeßgerät, eine Netzzuführung, Druckluftsteuerleitungen zur Temperatursteuerung durch den Wärmeaustauscher und ein Rechenteil.

Anhand der beigefügten Zeichnungen werden beispielhafte erfindungsgemäße Ausführungsformen dargestellt; es zeigt

Figur 1 eine schematische Darstellung der erfindungsgemäßen Vorrichtung und

Figur 2 eine schematische Darstellung des erfindungsgemäßen Steuergeräts.

Die erfindungsgemäße Vorrichtung besteht aus einem Tank 1, der mit Zuflußleitungen zur Zufuhr und Abnahme des Milchverarbeitungsrückstands sowie erforderlichen Ventilen, Pumpen usw. ausgerüstet ist. Der Vorratstank 1, der zugleich der Fermentationstank ist, kann irgendein beliebiger Tank sein, wie er in Milchverarbeitungsbetrieben, insbesondere Molkereien, vorhanden ist. Er soll mit einem Rührwerk ausgerüstet sein, welches langsam läuft, und er kann mit einem Mantel versehen sein und heizbar oder kühlbar sein. Der Tank ist über Leitungen 14 mit dem erfindungsgemäßen Steuergerät verbunden. In den Leitungen können Filter, Pumpen 15, Ventile, Wärmeaustauscher 16 vorgesehen sein.

Das Milchverarbeitungsprodukt wird im Kreislauf aus dem Vorratstank, gegebenenfalls durch den Wärmeaustauscher, durch das Steuergerät wieder in den Vorratstank geleitet.

In Figur 2 ist das erfindungsgemäße Steuergerät 2 schematisch dargestellt.

Das Steuergerät bzw. die Steuerungseinrichtung 2 weist ein Gehäuse 3 auf (wie in Figur 1 dargestellt). Es ist in sich abgeschlossen, kompakt gebaut und kann auf Rollen befestigt sein, so daß es leicht transportierbar ist. Durch das erfindungsgemäße Steuergerät 2 geht ein Durchlaufrohr 4 für die Milchverarbeitungsrückstände hindurch, welches an seinen Auslässen mit den üblichen genormten Armaturen der Milchindustrie versehen ist. Über diese Armaturen wird das Durchlaufrohr an seinen Enden mit den Leitungen, die zwischen dem Tank und dem Steuergerät vorgesehen sind, verbunden. Die Armaturen sind nicht in der Zeichnung dargestellt.

In dem Durchlaufrohr 4, durch das das Milchverarbeitungsprodukt strömt, sind zwei Vorrichtungen zur Temperaturmessung (Temperatursensoren 7, 8) und zwei Einrichtungen zur pH-Messung (pH-Sensoren 5 und 6) vorgesehen. Diese Einrichtungen zur Messung des pH-Werts und der Temperatur sind

über den Rechner 12 mit einer Pumpe 9 verbunden, durch die die Ammoniak- bzw. Ammoniakwasserzufuhr geregelt wird. Die Pumpe 9 ist durch die Leitung 11 für die Ammoniakzufuhr mit dem Durchlaufrohr für die Milchverarbeitungsrückstände verbunden, und über diese Leitung wird Ammoniak gezielt zu dem Milchverarbeitungsrückstand in Abhängigkeit von den Meßergebnissen zugegeben. Die Leitung 11 kann weiterhin mit einem Dosierungsventil 10 versehen sein, welches ebenfalls von der Rechenlage gesteuert wird. Die Steuereinrichtung umfaßt weiterhin eine Zuflußleitung 11 für das Ammoniak, die zu dem Vorratstank 7 für Ammoniak führt.

Der Durchfluß kann ein leichtes Gefälle zum Auslauf hin besitzen, und er weist weiterhin ein Schauglas 13 auf. Das Schauglas dient zur Überprüfung der Funktion. Der Einlauf für die Zufuhr von Ammoniakwasser ist so bemessen, daß ein Verhältnis zwischen Substratstrom (Einheit L/sec) und Ammoniakwasserzufuhr von 10 : 1 nicht unterschritten wird, wodurch eine sanfte Neutralisation erreicht wird.

Die Sensoren für die pH- und Temperaturmessung sind durch zweifache Skalen in dem Regelteil abzulesen. Bei Ausfall sowohl eines Sensors für die pH-Messung oder die Temperaturmessung kann der als Reserve vorhandene zweite Sensor die Prozeßführung übernehmen, wodurch eine Unterbrechung der Fermentation und ein Zusammenbruch des Verfahrens sicher verhindert werden können. Grundsätzlich ist es auch möglich, nur je einen Sensor für die Temperatur- und pH-Messung zu verwenden. Aus Sicherheitsgründen ist es jedoch bevorzugt, zwei Sensoren vorzusehen.

Durch entsprechende Steuerungselemente kann ein pH- und Temperaturbereich mit willkürlichen Bandbreiten von Hand eingestellt werden. Der pH-Bereich kann im Bereich von pH 3,0 bis 6,5 und der Temperaturbereich im Bereich von 18 bis 60° C liegen. Die jeweiligen Bereiche können jedoch auch auf einen pH-Wert von $\pm 0,2$ und eine Temperatur von $\pm 0,3$° C verengt werden. Es ist jedoch ein großer Vorteil, daß man bei dem erfindungsgemäßen Verfahren innerhalb weiter Temperatur- und pH-Bereiche arbeiten kann.

Der Rechenteil des Geräts kann so eingstellt werden, daß bis zum Verbrauch einer beliebigen Menge definierten Ammoniakwassers die kontinuierliche Teilneutralisation erfolgt. Anschließend erfolgt automatisch eine Endneutralisation auf einen wiederum im voraus festgelegten pH-Wert. Die verbrauchte Menge (Ammoniakwasser bzw. $NH_3$) wird optisch und durch Schreibgeräte registriert.

Das Gerät besitzt ferner ein Rechenteil, das die qualitative und quantitative Produktion von Milchsäure registriert, mit den pH- und Temperaturwerten vergleicht und durch Korrektur von pH und Temperatur den Prozeß so steuert, daß er in Richtung maximaler Milchsäureproduktion läuft, um den Fermentationsvorgang, abhängig von der eingesetzten Kultur, zu optimieren.

Durch das Steuergerät wird das Verfahren laufend korrigiert, und dadurch findet eine schnellere Milchsäurebildung und Ammonisierung statt, und insgesamt erhält man eine Zeitverkürzung. Alle Werte werden durch einen installierten Schreiber zeitgekoppelt notiert, so daß schließlich je nach Kultureinsatz Prozeßprogramme erarbeitet werden können. Diese können dann im Gerät gespeichert und abgerufen werden und für die Einleitung des Prozesses je nach Kultur und Substrat verwendet werden. Die mitlaufende Prozeßkorrektur, die dem Adaptionsvermögen im Verlauf der Fermentation Rechnung trägt, kann dabei in Betrieb gehalten oder abgekoppelt werden.

Das Gerät kann, bedingt durch die Abmessungen seiner Einrichtungen, in einer Ausführung für eine Substratvorlage von 15 000 bis 100 000 kg Substrat oder für eine Substratvorlage von 50 000 bis 250 000 kg Substrat gebaut werden. Es können auch Geräte, die für noch größere Substratvorlagen geeignet sind, gebaut werden.

Durch die erfindungsgemäße Steuereinrichtung ist es möglich, das normalerweise über eine Arbeitsschicht zeitlich hinausreichende Verfahren unabhängig von Personal oder Personalwechsel korrekt und ohne Beeinflussung von Hand aufrechtzuerhalten. Dies ist für die Wirtschaftlichkeit und Sicherheit ein wesentlicher Vorteil.

Im folgenden sind die Funktionen der einzelnen Einrichtungen der Steuereinrichtung 2 nochmals schematisch dargestellt.

| Einrichtungsteil | Ausführungsform | Beschreibung und Vorteile |
|---|---|---|
| Steuerungseinrichtung (2) | Gehäuse (3) in sich so geschlossen, daß es abgeschirmt ist, mobil, mit Anschlüssen für: | Verwendung beweglich, fahrbar, anschließbar an alle üblichen genormten Milcharmaturen, Stromstecker (Kabel), anschließbar an flexible Ammoniak(wasser)leitung aus Vorratsbehälter, |
| | Molkendurchlauf NM 65/+80/ Strom 220+240 V Wechselstrom | pneumatischer Ausgang zur Steuerung Wärmeaustauscher (Ventile) |
| | 3/4 Zoll Ammoniakwasser | warm/kalt |
| | Pneumatik f. Wärmeaustauscher | elektrischer Ausgang zur Steuerung von Pumpen (Drehstrom/kraft) |
| Durchlaufrohr für Milchverarbeitungsrückstände (4) | NW65/+80, eingebaut 2 pH-Sensoren (5, 6); 2 Temperatursensoren (7, 8); Schauglas (13), $NH_3$- bzw. Wasserzuführung (11) | in einem integrierten Durchlaufteil werden die benötigten Parameter (°C+pH) abgenommen, auf Skalen angezeigt, im Rechenteil übernommen und ausgewertet, doppelte Reserve pH, Temp. zum Schutz des Prozesses gegen Ausfall+Pannen, Sichtbeobachtung des Prozesses, im Durchlauf wird die Ammonisierung vorgenommen (Zuleitung von Dosiereinheit für $NH_3$) |
| pH-Messung | 2 Sensoren (5, 6)+2 Sichtskalen, Rechenteil, Bandbreiteneinstellung | pH-Bereich 3,0—6,5, Meßbereich jedoch weiter, Einstellung im pH-Bereich auf steuerbare pH-Bänder, beliebig zu verengen bis auf ±0,2 pH, Sensoren wählbar und austauschbar während des Prozesses=Absicherung gegen Ausfall |
| Temp.-Messung | 2 Sensoren (7, 8)+2 Sichtskalen, Rechenteil, Bandbreiteneinstellung | Temp.-Bereich 18—60°C, Meßbereich jedoch weiter, Einstellung im Temp.-Ber. auf steuerbare Temperaturbänder, beliebig zu verengen bis auf ±0,2°C, Sensoren wählbar und austauschbar während des Prozesses=Absicherung gegen Ausfall |
| Ammonisierung | Dosierpumpe (9) 1—3000 ml/min, Mengensensor, Rechenwerk, Schreibwerk, Mengenvorgabe | einstellbare, beliebig variable Fördermenge, einstellbar auf Substrat und Kultur bzw. Milchsäureproduktion zur Neutralisation, sanfte Neutralisierung=Schonung der Kultur, des Substrats und der Reaktion gegen empfindliche Substratbestandteile |
| Mengenvorgabe | beliebig wählbare, einstellbare $NH_3$-Zudosierungsmenge, separat Zählung der Menge d. Endneutralisierung auf pH-Endwerte | substratbezogene, quantitative Vorgabe der Menge $NH_3$ (Wasser) zum Eiweißaufbau als AL während der Fermentation, nach Verbrauch Meldesignal und automatische Nachneutralisierung auf einen festgelegten pH (bevorzugt 5,8) |

(Fortsetzung)

| Einrichtungsteil | Ausführungsform | Beschreibung und Vorteile |
|---|---|---|
| Rechenteil (12) | im Gerät integriert, Speicherteile, Rechenteil, Steuerteil | das Rechenteil zählt den $NH_3$-Verbrauch der Vorgabe, subtrahiert bis zu Erreichung von 0, hält Vorgabewert fest, neutralisiert dann nach, hält diesen Verbrauch ebenfalls fest; es speichert den pH und Temp.-Verlauf, vergleicht beides mit dem Verbrauch von $NH_3$ und geht daran, selbständig den pH- und Temp.-Bereich der maximalen Milchsäureerzeugung = des maximalen $NH_3$-Verbrauchs aufzusuchen; es speichert eingegebene Daten für verschiedene Kulturtypen für ein darauf abgestimmtes Programm, so daß die Auswahl für die Kultur gewählt, eingestellt und automatisch gesteuert werden kann |
| Pumpenteil (15) | Intervallsteuerung mit Aussetzung, wenn Optimierung nicht erreicht | der Pump-Kreislauf läuft in steuerbaren Intervallen ab und wird bei Werten innerhalb der Bandbreiten pH + Temp. selbsttätig abgestellt. Arbeitet das Gerät noch am Erreichen der optimalen Wertvorgaben oder der automatischen Korrektur auf Fermentationsintensität, so setzt das Abschalten so lange aus, bis dieser Zustand erreicht ist, damit wird elektrischer Energieverbrauch minimiert |
| Gerät (2) | das Steuerungsgerät ist anpaßbar an die betrieblich gegebenen Verhältnisse durch Substrat, Kultur, Volumen und Versorgungsmöglichkeiten | praxisorientierte Flexibilität der Prozeßführung an alle betriebsgegebenen Situationen |
| Schreiber | hält in der Zeit die Meßwerte (pH, Temp. $NH_3$-Zugabe) und Mengenwerte fest und zeichnet sie auf | wissenschaftliche und praktische Auswertung des Prozeßverlaufs hinsichtlich aller relevanten Parameter |

Erfindungsgemäß ist es möglich, je nach Substrat und praktischer Lage des Betriebs hinsichtlich seiner Technologie, Kulturen in Mischungen im normalen, mesophilen und thermophilen Bereich optimal zu nutzen. Die doppelte Anpassungsfähigkeit, einmal durch die eingesetzten Mischkulturen, zum anderen durch die flexible Einstellungsmöglichkeit des Geräts (hinsichtlich pH und Temperatur), bietet eine umfassende Absicherung des Prozeßverlaufs, wodurch die Produktion ohne zusätzlichen Personaleinsatz wesentlich gesteigert wird.

Zur Vermeidung von starken pH-Schwankungen ist es wesentlich, daß die Neutralisation langsam erfolgt. Dies wird durch die spezielle Dosiereinrichtung 9, 12 erreicht. Als spezielle Dosiereinrichtung kann man eine Dosierpumpe oder auch ein Dosierventil verwenden. Durch die Dosiereinrichtung erfolgt die Zufuhr von Ammoniak oder Ammoniakwasser im Bereich von 1 ml bis 3000 ml/min, und dadurch wird eine starke Bräunung des Substrats weitgehend vermieden. Ebenso wird eine Schädigung von nativen Aminosäuren oder Proteinen, die in den Rückständen vorhanden sind, vermieden. Solche Aminosäuren oder Proteine ändern sich, wenn sie starken pH-Änderungen ausgesetzt sind.

Aus Gründen der Energieeinsparung und der Schonung der Kultur durch zu starken mechanischen, thermischen oder chemischen Verschleiß kann die Anlage so geschaltet werden, daß der Kreislauf des Umpumpens willkürlich nur in bestimmten Zeitintervallen zwischen 1 und 20 min erfolgt und daß dann die Anlage für eine bestimmte Zeit abgestellt wird. Die Schaltung kann dabei so ausgelegt werden, daß sich der Kreislauf bei unverändertem pH oder unveränderter Temperatur sofort wieder abstellt, während bei intensiver Tätigkeit durch pH-Erniedrigung, was einer Milchsäurebildung entspricht, und entsprechend verstärkter Zufuhr von Ammoniak oder Ammoniakwasser die Abschaltung so lange ausgesetzt wird, bis das erwünschte optimale Gleichgewicht wieder erreicht worden ist.

Es ist bevorzugt, daß das Endprodukt einen pH-Wert von 5,8 besitzt. Dieser Bereich hat sich für den Geschmack und die Stabilität bei der Lagerung als besonders wichtig erwiesen. In diesem Bereich stabilisiert der schwach saure pH das Ammoniumlactat gegen Abspaltung von $NH_3$.

Bei dem erfindungsgemäßen Verfahren wird die Fermentation dann abgebrochen, wenn die vorgegebene Menge an Ammoniak oder Ammoniakwasser verbraucht ist. Das erfindungsgemäße Steuergerät löst dann die abschließende Neutralisation auf pH 5,8 aus und vermerkt die hierbei benötigten, jedoch praktisch unterschiedlichen und zusätzlichen Verbrauch von Ammoniak bzw. Ammoniakwasser bis zu diesem End-pH-Wert. Beide Menge werden vom Gerät aufgezeichnet.

Bei dem erfindungsgemäßen Verfahren erhält man ein Ammoniumlactat enthaltendes Produkt. Dieses Produkt ist besonders als Tierfutter oder Tierfutterbestandteil geeignet. In durchgeführten Fütterungsversuchen hat sich erwiesen, daß so gewonnene ammonisierte Milchrückstände in ihrer Trockenmasse bis zu 80% Proteinwert aus Rest an nativem Milcheiweiß und Eiweißwert von Ammoniumlactat bestehen. Es kann jedoch ein beliebig zu wählender niedrigerer Eiweißgehalt vorprogrammiert, erstellt und erreicht werden. Damit ist es möglich, für die Praxis ein optimales Futtermittel zur Verfügung zu stellen.

Die folgenden Beispiele erläutern die Erfindung.

## Beispiel 1

### Allgemeines Verfahren

Das Steuergerät, welches transportabel ist, wird mit dem stationären Vorratstank für das Milchkoppelprodukt, gegebenenfalls über Wärmeaustauscher, verbunden. Mit dem Steuergerät wird weiterhin der Vorratstank für Ammoniak oder Ammoniakwasser verbunden, und das Steuergerät wird an den Strom und die Pneumatik angeschlossen.

Danach wird der Vorratsbehälter so weit gefüllt, daß der Prozeß in Gang gehalten werden kann, d. h. es wird ca. $^1/_5$ der vollständigen Menge zugegeben. Dann werden 0,5 bis maximal 1,6% flüssige Betriebskultur, bezogen auf 100% Fermentationsvolumen im Vorrats- bzw. Fermentationstank, zugefügt, und das Steuergerät wird in Abhängigkeit von der Art der Kultur und dem Tankinhalt programmiert.

Es werden folgende Werte eingestellt:

a) vorausberechnete $NH_3$-Menge für das Gesamtvolumen,
b) Temperaturbereich,
c) pH-Bereich,
d) Intervalleinstellung für die Kreislaufpumpe.

Das Verfahren wird gestartet, wobei anschließend das Volumen an zulaufenden Koppelprodukten im laufenden Prozeß erhöht werden kann.

Das Verfahren läuft ab, wobei der pH-Wert, die Temperaturwerte, der $NH_3$-Verbrauch dauernd angezeigt werden. Man läßt das Verfahren ablaufen, bis die vorausberechnete Menge an $NH_3$ bzw. $NH_3$-Wasser verbraucht ist.

Bei Einstellung der Automatik werden die Werte gespeichert und vom Gerät so ausgewertet, daß in Richtung der schnellsten und quantitativ höchsten Milchsäureproduktion gefahren wird. Die Milchsäureproduktion hängt von der verbrauchten $NH_3$-Menge ab. Dies wird durch eine selbsttätige Beeinflussung des zu erreichenden pH- und Temperaturwerts erreicht. Die Ergebnisse werden jeweils durch den Schreiber aufgezeichnet.

Nach dem Verbrauch der vorausberechneten Menge an $NH_3$ erfolgt die Endneutralisation auf den zuvor bestimmten pH-Wert (vorzugsweise 5,8).

Die Fermentationsflüssigkeit wird dann abgepumpt und konzentriert. Ein Rest der Fermentationsflüssigkeit kann im Vorratsbehälter verbleiben und zum Impfen der nächsten Charge dienen. Die Fermentationsflüssigkeit enthält jetzt adaptierte Kulturen. Gegebenenfalls kann man durch neue Kulturzugabe auffrischen.

Beispiel 2

Spezielles Ausführungsbeispiel

In einen Vorratstank werden 100 000 l Milchrückstände eingegeben, welche sich wie folgt zusammensetzen:

| Menge (l) | Masse (kg) | Art des Milchrückstands bzw. der Koppelprodukte | Parameter TS | RP | MZ | MS |
|---|---|---|---|---|---|---|
| 40 000 | 41 000 | Nachprodukt von Speisequark/Sauermilchkäseprod. | 6,3 | 11 | 68 | 7 |
| 40 000 | 41 000 | Nachprodukt aus Thermoquarkproduktion | 6,0 | 8 | 70 | 5 |
| 20 000 | 20 400 | Permeat aus Ultrafiltration | 5,3 | 6 | 82 | 2 |
| 100 000 | 102 400 | | 6 | 8,9 | 71 | 5,3 |

Inhaltsstoffe in kg:

| TS | RP | MZ | Mi-S |
|---|---|---|---|
| 2583 | 284 | 1756 | 181 |
| 2460 | 197 | 1722 | 123 |
| 1060 | 64 | 869 | 21 |
| 6103 | 545 | 4347 | 325 |

TS = Trockenmasse
RP = Rohprotein
MZ = Milchzucker
MiS = Milchsäure

Die Berechnung des Verbrauchs an $NH_3$ erfolgt anhand der Menge an Milchzucker und Milchsäure in der Trockenmasse und durch Umrechnung auf Protein.

Zur Bildung von Ammoniumlactat vorhanden:

| | |
|---|---|
| MZ | 4347 kg |
| MiS | 325 kg |
| | 4672 kg |

Ankopplung von $NH_3$ zu Ammoniumlactat (=AL) nach Molgewicht = 107 aus 90 Molgewicht Lactat/Mi-S zu 17 Molgewicht $NH_3$ aus Ammoniakwasser (AW) ergibt theoretische Bildung von:

| | | |
|---|---|---|
| 4672 : 90 = 51,91 × 17 = | | 882 kg |
| | AL | 5554 kg |
| N-Anteil in AL × 6,25 entspricht | | |
| Eiweißwert | RP/AL | 4540 kg |
| dazu natives Eiweiß aus Koppelprodukten | RP/nat | 545 kg |
| | RP/ges. | 5085 kg |

Abschätzung der Veränderung in der Gesamtmenge, wenn 882 kg $NH_3$ durch Ammoniaklösung 25%ig mit spez-Gew. 0,910 zugegeben werden

| | | | | |
|---|---|---|---|---|
| 3'877 | 3'528 | Parameter s. oben | | MZ + Mi-S = Lactat |
| 104 Tsd. | 105'928 | Fermentationsprod. 6985 5085 Änderung der TS 6,6 72—73 | 4672 | |

Durch das erfindungsgemäße Verfahren wird die Trockenmasse um fast 10% erhöht, der Eiweißwert als Protein auf über 70% in der Trockenmasse gesteigert, wobei das Verhältnis zwischen nativem Protein und Protein aus Ammoniumlactat etwa 1 : 8 beträgt.

Die Endneutralisation auf pH 5,8 benötigt etwa $^{1}/_{10}$ der hier berechneten Menge $NH_3$ und verbleibt (als Verlust) unberücksichtigt.

## Patentansprüche

1. Verfahren zur Herstellung von Ammoniumlactat enthaltenden Mischungen aus wäßrigen Milchverarbeitungsrückständen durch Zugabe zu den Milchverarbeitungsrückständen oder Gemengen, die Milchsäure und/oder Lactose und Milchsäurebildner enthalten oder zu denen zusätzlich Milchsäurebildner zugegeben worden sind, ohne weitere Vorbehandlung, gegebenenfalls unter Erwärmen oder Kühlen und Bewegen, Ammoniak in solcher Rate, daß der pH-Wert unter 6,0 gehalten wird, und Zugabe von Ammoniak bis zu einem pH-Wert von höchstens 6,5, nachdem die gewünschte Menge Ammoniumlactat gebildet worden ist, und gegebenenfalls anschließende Konzentrierung des erhaltenen Reaktionsgemisches in an sich bekannter Weise bis auf einen Feststoffgehalt von maximal 85%, dadurch gekennzeichnet, daß man als Milchsäurebildner Mischkulturen verwendet.

2. Verfahren nach Anspruch 1, dadurch gekennzeichnet, daß man eine normale, mesophile oder thermophile Mischkultur verwendet, die mindestens eine Kultur, die im oberen pH-Bereich wirkt, und mindestens eine Kultur, die im unteren pH-Bereich wirkt, enthält.

3. Verfahren nach Anspruch 1 oder 2, dadurch gekennzeichnet, daß man Streptokokken, wie Str. lactis, Str. faecalis, Str. salivarius, Str. cremoris, Str. thermophilus, Str. diacetylis, Str. faecium oder ihre Gemische, als Kulturen, die im oberen pH-Bereich wirken, verwendet.

4. Verfahren nach Anspruch 1 oder 2, dadurch gekennzeichnet, daß man Stäbchenbakterien und Langstäbchenbakterien, wie Lbc. bulgaricus, Lbc. lactis, Lbc. helveticus, Lbc. acidophilus und Lbc. delbrückii oder ihre Gemische, als Kulturen, die im unteren pH-Bereich wirken, verwendet.

5. Verfahren nach Anspruch 1, 2, 3 oder 4, dadurch gekennzeichnet, daß man eine Mischkultur verwendet, die Leuconostoc-Arten (mesenteroides, cremoris), Streptobacter, Betabakterien und Lbc. fermentum und viridescens, Bifidobacterium bifidus, Propionobacterium oder ihre Gemische enthält.

6. Verfahren nach mindestens einem der Ansprüche 1 bis 5, dadurch gekennzeichnet, daß man als Milchverarbeitungsrückstand Süßmolke, milchsaure Molke aus Speisequark oder Frischkäse, Molke aus Thermoquark, enteiweißte Molken aus der Membranabtrennung, entzuckerte Molken aus der Lactoseproduktion, Salzwasser aus der Elektrodialyse, Permeate, Milchzuckerwasser aus der Lactoseproduktion, Buttermilch oder Rückstände, die bei der Milchzerlegung mit Hilfe moderner Verfahren, wie zum Beispiel Ultrafiltration, Umkehrosmose, Elektrodialyse, anfallen, oder ihre Gemische verwendet.

7. Verfahren nach einem der vorherigen Ansprüche, dadurch gekennzeichnet, daß die Konzentration des Reaktionsgemisches auf einen Feststoffgehalt von 35 bis 80% erfolgt.

8. Vorrichtung zur Durchführung des Verfahrens nach einem der Ansprüche 1 bis 7, gekennzeichnet durch einen Tank (1), eine Steuerungseinrichtung (2), Leitungen (14) zwischen dem Tank und dem Steuergerät, Pumpen (15), einem Wärmeaustauscher (16), einem Ammoniakvorratstank (17), Leitungen (11) zur Ammoniakzufuhr, eine Netzzuführung (18) und Druckluftsteuerleitungen (19, 20) für den Wärmeaustauscher.

9. Steuerungseinrichtung gemäß Anspruch 9, gekennzeichnet durch ein Gehäuse (3), ein Durchlaufrohr (4) für die Milchverarbeitungsrückstände mit einem Schauglas (13), zwei Einrichtungen (5, 6) zur pH-Messung, zwei Einrichtungen (7, 8) zur Temperaturmessung, eine Dosierpumpe (9), eine Zufuhrleitung (11) für Ammoniak mit einem Durchflußmeßgerät (10), eine Netzzuführung (18), Druckluftsteuerleitungen (19, 20) zur Temperatursteuerung durch den Wärmeaustauscher und ein Rechenteil (12).

## Claims

1. A process for the production of mixtures containing ammonium lactate from aqueous milk processing residues by adding ammonia to the milk processing residues or mixtures, which contain lactic acid and/or lactose and lactic acid generators or to which lactic acid generators have been additionally added, without further pretreatment, optionally with heating or cooling and agitation, at such a rate that the pH-value is kept below 6.0 and adding ammonia to a pH-value of at most 6.5 after the desired quantity of ammonium lactate has been formed, optionally followed by concentration of the reaction mixture obtained in known manner to a solids content of at most 85%, characterized in that mixed cultures are used as the lactic acid generators.

2. A process as claimed in Claim 1, characterized in that a normal, mesophilic or thermophilic mixed culture containing at least one culture acting in the uppper pH-range and at least one culture acting in the lower pH-range is used.

3. A process as claimed in Claim 1 or 2, characterized in that streptococci, such as Str. lactis, Str. faecalis, Str. salivarius, Str. cremoris, Str. thermophilus, Str. diacetylis, Str. faecium, or mixtures thereof are used as the cultures acting in the upper pH-range.

4. A process as claimed in Claim 1 or 2, characterized in that bacillus and lactobacillus species, such as Lbc. bulgaricus, Lbc. lactis, Lbc. helveticus, Lbc. acidophilus and Lbc. delbrückii, or mixtures thereof are used as the cultures acting in the lower pH-range.

5. A process as claimed in Claim 1, 2, 3 or 4, characterized in that a mixture containing leuconostoc species (mesenteroides, cremoris), streptobacter, beta-bacteria and Lbc. fermentum and viridescens, Bifidobacterium bifidus, propionobacterium or mixtures thereof is used.

6. A process as claimed in at least one of Claims 1 to 5, characterized in that sweet whey, lactic acid whey from curd cheese or fresh cheese, whey from thermocurds, deproteinized whey from membrane separation, desugared whey from lactose production, saltwater from electrodialysis, permeates, lactose water from lactose production, buttermilk or residues of the type obtained in the decomposition of milk by modern techniques, such as for example ultrafiltration, reverse osmosis, electrodialysis, or mixtures thereof are used as the milk processing residue.

7. A process as claimed in any of the preceding Claims, characterized in that the reaction mixture is concentrated to a solids content of from 35 to 80%.

8. An apparatus for carrying out the process claimed in any of Claims 1 to 7, characterized by a tank (1), a control unit (2), pipes (14) between the tank and the control unit, pumps (15), a heat exchanger (16), an ammonia reservoir (17), ammonia feed pipes (11), a mains lead (18) and compressed-air control lines (19, 20) for the heat exchanger.

9. A control unit as claimed in Claim 9, characterized by a housing (3), a throughflow pipe (4) for the milk processing residues with an inspection window (13), two pH meters (5, 6), two temperatures gauges (7, 8), a metering pump (9), an ammonia feed pipe (11) with a throughflow meter (10), a mains lead (18), compressed-air control lines (19, 20) for temperature control through the heat exchanger and a computer (12).

## Revendications

1. Proceédé pour la préparation de mélanges contenant du lactate d'ammonium en partant de résidus aqueux de la transformation du lait par addition, aux résidus de la transformation du lait ou à des mélanges qui contiennent de l'acide lactique et/ou du lactose et des formateurs d'acide lactique ou auxquels on a ajouté en outre des formateurs d'acide lactique, sans autre pré-tratitement, éventuellement avec chauffage ou refroidissement et mouvement, d'ammoniac à un débit tel quel pH soit maintenu en dessous de 6,0 et addition d'ammoniac jusqu'à un pH de 6,5 au maximum une fois que la quantitité dásirée de lactate d'ammonium a été formée et éventuellement ensuite concentration du mélange réactionnel obtenu, de manière en soi connue, jusqu'à une teneur en solides de 85% au maximum, caractérisé en ce qu'on utilise des cultures mixtes en tant que formateurs d'acide lactique.

2. Procédé selon la revendication 1, caractérisé en ce que l'on utilise une culture mixte normale, mésophile ou thermophile qui contient au moins une culture qui agit dans la gamme supérieure de pH et au moins une culture qui agit dans la gamme inférieure de pH.

3. Procédé selon la revendication 1 ou la revendication 2, caractérisé en ce que l'on utilise, comme cultures qui agissent dans la gamme supérieure de pH, des streptocoques comme Str. lactis, Str. faecalis, Str. salivarius, Str. cremoris, Str. thermophilus, Str. diacetylis, Str. faecium, ou leurs mélanges.

4. Prodédé selon la revendication 1 ou la revendication 2 en ce que comme cultures qui agissent dans la gamme inférieure de pH, on utilise des bactéries en bâctonnet et des bactêries en bâtonnet long, comme Lbc. bulgaricus, Lbc. lactis, Lbc. helveticus, Lbc. acidophilus et Lbc. delbrückii ou leurs mélanges.

5. Procédé selon les revendications 1, 2, 3 ou 4, cractérisé en ce que l'on utilise une culture mixte qui contient des espèces de Leuconostoc (mesenteroides, cremoris) des Streptobacter, des bactéries $\beta$ et

Lbc. fermentum et viridescens, Bifidobacterium bifidus, Propionobacterium ou leurs mélanges.

6. Procédé selon au moins une des revendications 1 à 5, caractérisé en ce que, comme résidu de la transformation du lait, on utilise le sérum doux, le sérum contenant de l'acide lactique et tiré de la caillebotte ou du fromage frais, des sérums déprotéinés provenant de la séparation de la membrane, des sérums désucrés provenant de la fabrication du lactose, de l'eau salée provenant de l'électrodialyse, des perméats, de l'eau lactosée provenant de la fabrication du lactose, du babeurre ou des résidus qui sont obtenus lors de la décomposition du lait à l'aide de procédés modernes, comme par exemple l'ultrafiltration, l'osmose inverse, l'électrodialyse, ou leurs mélanges.

7. Procédé selon l'une des revendications précédentes, caractérisé en ce que la concentration du mélange réactionnel s'effectue jusqu'à une teneur en solides de 35 à 80%.

8. Dispositif pour la mise en oeuvre du procédé selon l'une des revendications 1 à 7, caractérisé par un réservoir (1), un dispositif de commande (2), des tuyaux (14) entre le réservoir et le dispositif de commande (2), des tuyaux (14) entre le réservoir et le dispositif de commande, des pompes (15), un échangeur de chaleur (16), un réservoir à ammoniac (17) des tuyaux (11) pour l'amenée d'ammoniac, une arrivée de réseau (18) et des tuyaux de commande d'air comprimé (19, 20) pour l'échangeur de chaleur.

9. Dispositif de commande selon la revendication 9, caractérisé par une enveloppe (3), un tube de passage (4) pour les résidus de la transformation du lait, muni d'un verre de regard (13), deux dispositifs (5, 6) pour la mesure du pH, deux dispositifs (7, 8) pour la mesure de la température, une pompe doseuse (9), un tuyau d'amenée (11) pour l'ammoniac muni d'un débitmètre (10), une arrivée de réseau (18), des tuyaux de commande d'air comprimé (19, 20) pour la commande de température par l'échangeur de chaleur et une partie de calcul (12).

Fig. 1

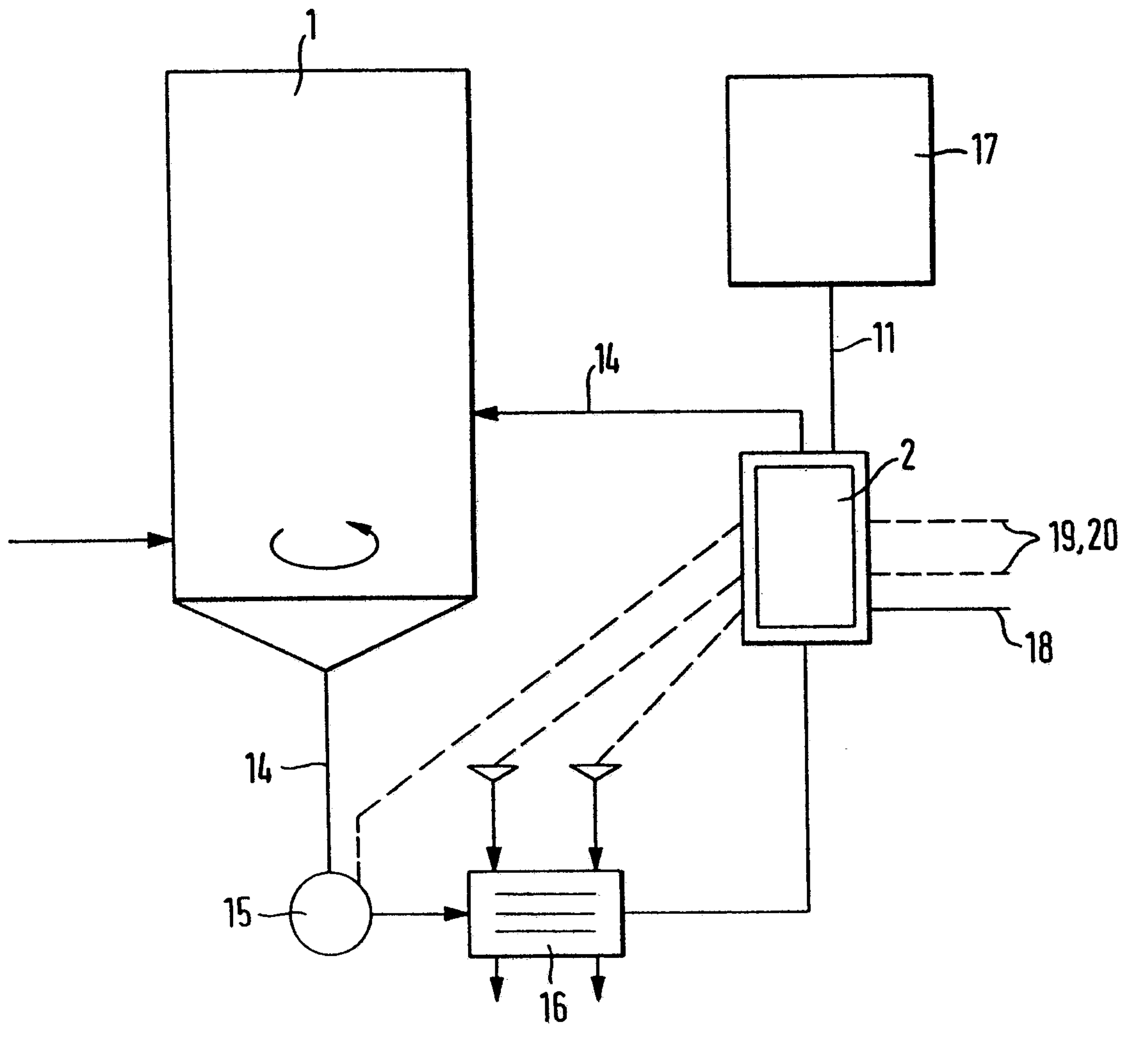

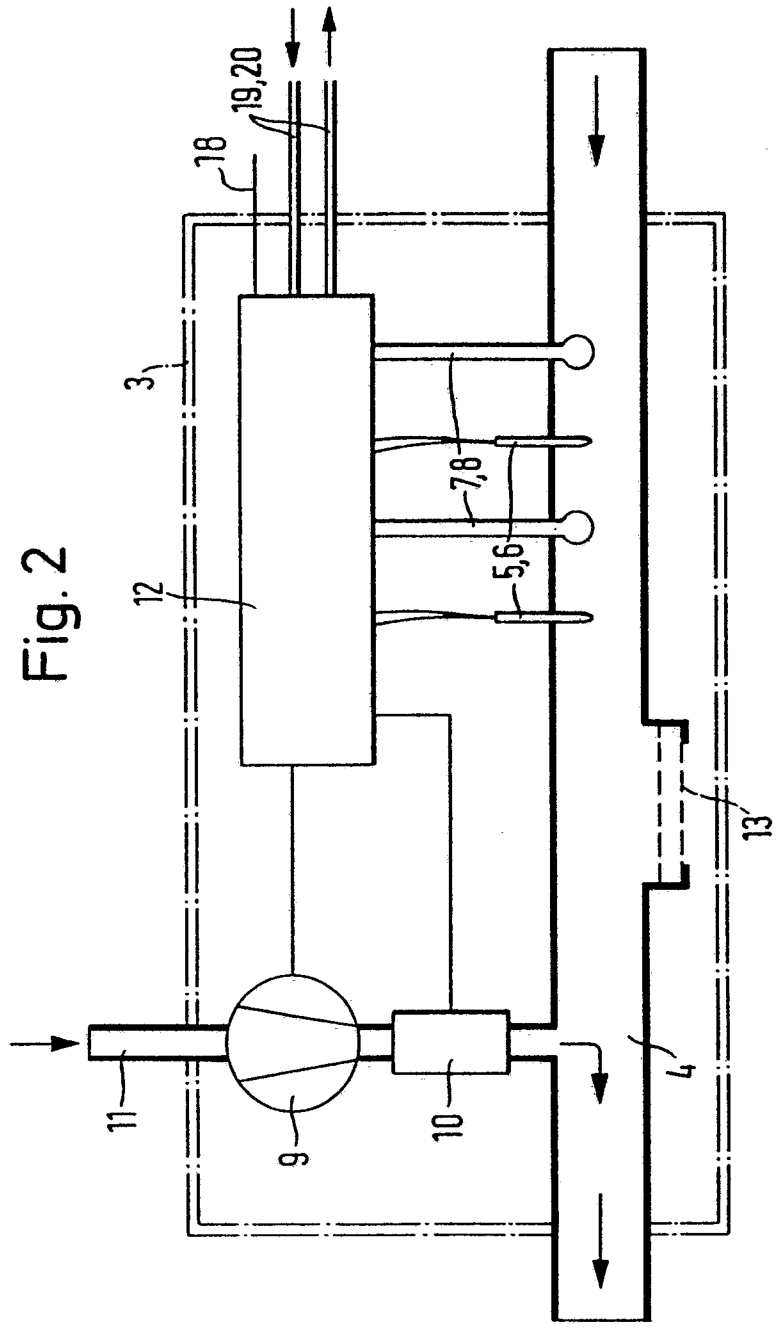


Fig. 2